# EUROPEAN PATENT SPECIFICATION

(11) **EP 2 598 019 B1**
(45) Date of publication and mention of the grant of the patent: **05.09.2018**
(21) Application number: 11813295.0
(22) Date of filing: 29.07.2011
(51) Int. Cl.: A61B 1/317, A61B 1/00, A61B 1/05

(54) **ARTHROSCOPIC SYSTEM**
ARTHROSKOPISCHES SYSTEM
SYSTÈME ARTHROSCOPIQUE

(30) Priority: 29.07.2010 US 846747; 12.05.2011 US 201113106078
(43) Date of publication of application: 05.06.2013
(73) Proprietor: Cannuflow, Inc., Campbell, CA 95008 (US)
(72) Inventor: KUCKLICK, Theodore, R., San Jose CA 95110 (US)
(74) Representative: Schiweck Weinzierl Koch Patentanwälte Partnerschaft mbB
(86) International application number: PCT/US2011/046030
(87) International publication number: WO 2012/016224

(56) References cited:
- US-A1- 2003 032 863
- US-A1- 2004 236 183
- US-A1- 2009 012 362
- US-A1- 2009 082 628
- US-A1- 2009 187 072
- US-A1- 2009 240 109
- US-B1- 6 379 347
- US-B2- 7 553 278

## Description

### Field of the Inventions

The inventions described below relate to the field of arthroscopic surgical instruments.

### Background of the Inventions

Arthroscopic surgery involves using optical instruments, such as an arthroscope, to visualize an operating field inside or near a joint of a patient. The same instrument or other instruments may be used to perform a surgical procedure in the operating field.

Known inflow and outflow arthroscope systems generally consist of several elements, which include a flexible or rigid tube, a light that illuminates the area the doctor wants to examine (where the light is typically outside of the body and delivered via an optical fiber system), a lens system that transmits an image to the viewer from the arthroscope and another channel that allows the entry of medical instruments or manipulators. The lens systems typically use pre-manufactured square or rectangular shaped CCD chips. Traditionally, arthroscopes are circular so that the arthroscope does not have sharp edges that may cause trauma to tissue. When the chips are housed within the arthroscope, this results in a great amount of wasted space between the square chips and the circular arthroscope that houses the chips.

As an example, US 2009/0187072 A1 discloses an endoscope including a rigid section having opposed first and second ends and an opening situated between the first and second ends, the rigid section defining a longitudinal axis; a handle portion coupled to a first end of the rigid section and having first and second scissor-type handles suitable for grasping by a user; and a base part situated at the second end of the rigid section and coupled to the first handle of the scissor-type handles such that displacement of the first handle causes a rotation of the base part.

In addition, current arthroscopes that use metal sheaths are prone to damage if the metal is touched with an RF wand. This stray RF current can damage the lens of the arthroscope or damage an imaging chip.

### Summary

The devices and methods described below provide for an arthroscope having square or rectangular lateral cross section herein after referred to as a rectangle or rectangular. The arthroscope can be used in an arthroscopic system that also includes a scope sheath that is matched to the dimensions of the arthroscope. The system includes a flow system, which sends fluid out of the end of the endoscope and brings debris and other fluid behind the field of view, thus allowing the surgeon to have a clear field of view while using the system.

This architecture allows the arthroscope to have a low profile thus making it less traumatic once introduced into anatomic spaces. Further, configuring the arthroscopic cross-section into the shape of the pre-manufactured CCD chip image configurations reduces costs associated with the manufacture of the scope.

To enhance the effectiveness of the systems, the arthroscope may include a protective cap for use over the distal tip of the arthroscope. The cap is constructed of a non-conductive polymer. The portion of the cap that covers a view port on the arthroscope is transparent. The cap protects the distal end of the arthroscope from accidental damage and also emits light from the tip of the arthroscope. The cap contains two tubes or pipes that terminate in openings on the cap. The cap also contains LEDs positioned on the proximal end of the tubes or pipes. Positioning the LEDs at the proximal end of the tubes or pipes provides the advantage that the imaging chip does not require a ring of LEDs around the cap and thus provides more space for the imaging chip which advantageously increases the space available for the imaging chip. It also provides the advantage that the imaging chips can allow for high light sensitivity with low illumination requirement from the LEDs. Also, the position of the LEDs allows irrigation fluid passing through the inflow channel to cool off the imaging chips and imaging sensor, resulting in less image noise. In addition, because the cap is constructed of a non-conductive polymer, the imaging chip is isolated and therefore protected from stray RF current.

The device provides for variable view angles in a single endoscope. The most common angles of view in an endoscope are 0 degrees (cystoscopy) 30 degrees (the most common arthroscope view angle) and 70 degrees (for difficult to visualize areas). A range of view angles may be produced in one scope, eliminating the need for having multiple view angle scopes in stock, or needing to remove and replace the scope to switch viewing angles. One way to achieve this is to deflect the chip view plane to the desired angle, by means of, for example, a pushrod. In another embodiment, two or more cameras are mounted to an angled face and the user selects a camera corresponding to the desired viewing angle.

The digital scope embodiments lend themselves to the ability to bend the scope shaft to gain access to anatomy not accessible with a straight rigid scope. Because of the architecture of the scope, the endoscope has the ability to have a variable angle of view, with the scope shaft bent at an angle. The angle of the shaft may either be pre-molded and fixed, or malleable and the bend angle and radius may be selected by the user.

### Brief Description of the Drawings

Figure 1 shows an arthroscope having a sheath that encloses an elongated core that has a square radial cross section, the elongated core has an imaging element on the distal end.
Figure 2 illustrates a cross-sectional view along Line A-A of Figure 1.
Figure 3 illustrates an arthroscope having an optical cap.
Figure 4 illustrates the features of the arthroscope pulled apart.
Figures 5a and 5b illustrate the elongated core of the arthroscope before it is folded into its final configuration.
Figure 6 illustrates the elongated core of the arthroscope in its final folded configuration.
Figures 7a and 7b illustrate another elongated core before it is folded into its final configuration.
Figure 8 illustrates another elongated core configuration.
Figure 9 illustrates an elongated core with a square tube or solid mandrel for additional rigidity.
Figure 10 illustrates a method of performing arthroscopic surgery on a patient using an arthroscope containing an elongated core with a square radial cross section.
Figure 11 illustrates an arthroscope where the fluid management is contained in a grommet-type cannula.
Figure 12 illustrates an arthroscope that can be used without requiring a user to hold it, providing the user the opportunity to use the arthroscope hands free.
Figure 13 illustrates an arthroscope with a molded optical cap and 3-D positioning sensors.
Figure 14 illustrates an optical cap for use with an arthroscope system and its features.
Figure 15 illustrates a side view of the optical cap.
Figure 16 illustrates an arthroscope having a variable view angle.
Figure 17 illustrates the distal end of the arthroscope of Figure 16.
Figure 18 illustrates the distal tip of the arthroscope of Figure 16.
Figure 19 illustrates a top view of Figure 18.
Figure 20 illustrates a cross-sectional view of the distal tip shown in Figure 18.
Figures 21 and 22 illustrate a nitinol mechanism for deflecting the camera.
Figure 23 illustrates an arthroscope having a solid state variable view angle.
Figure 24 is an exploded view of the distal tip of the arthroscope of Figure 23.
Figure 25 is an exploded cross-section view of Figure 23.
Figures 26 and 27 illustrates an arthroscope with an angled shaft.
Figure 28 illustrates a cross section view of Figure 27.

### Detailed Description of the Inventions

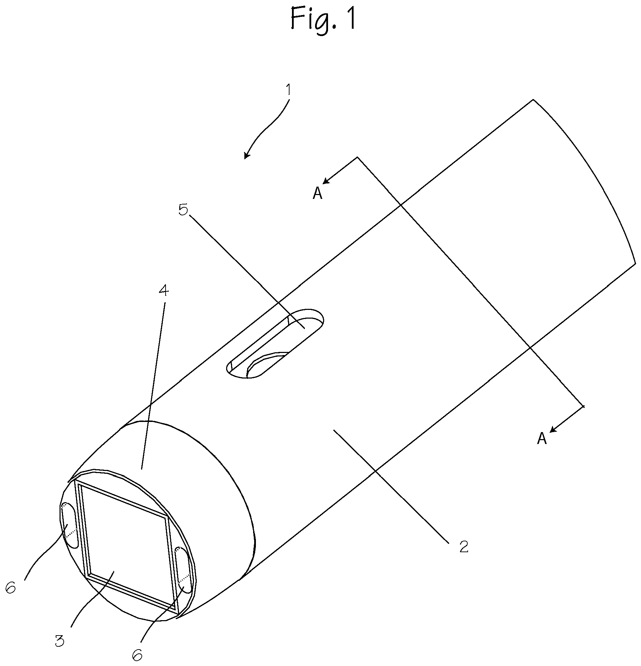

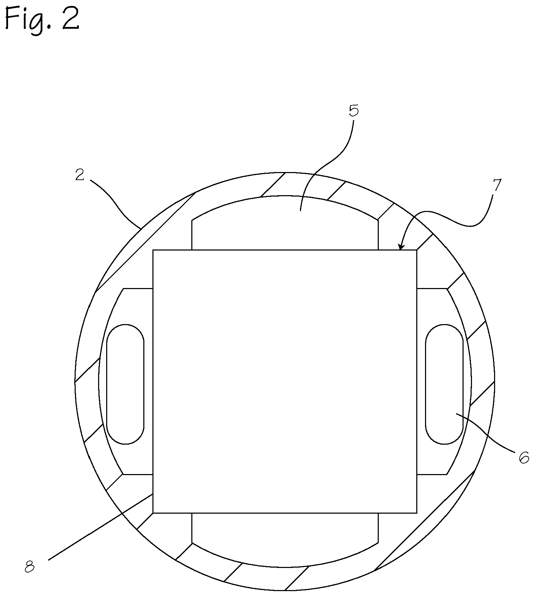

Figure 1 shows an arthroscope 1 having a sheath that encloses an elongated core having a square radial cross section (see Figure 2). Contained centrally within a sheath 2, the elongated core has a square imaging chip 3 located at the distal end of the elongated core. The elongated core and the imaging chip together form the imaging core of the arthroscope. An atraumatic tip 4 at the distal end may also encase the imaging chip. The elongated core has a square radial cross section that allows for the largest possible rectangular chip image package to be used in combination with the smallest possible round fluid sheath outside diameter. This combination allows a clear pocket flow system, which sends fluid out of the end of the arthroscope and brings debris and blood behind the operator's field of view. The system contains fluid outflow 5 and fluid inflow channels **6.** These channels are defined by the space created between the elongated core and the circular sheath surrounding it.

Figure 2 illustrates a cross-sectional view along Line A-A of Figure 1. Fluid enters the inflow channels 6 and flows axially into the joints. Fluid exits through the outflow channels 5 and comes behind the distal end of the arthroscopic sheath system and pulls blood and debris behind the field of view of the user. The fluid flow is perpendicular to the system creating a pocket of clear fluid in front of the system where it is needed the most. An elongated core having square radial cross section **7** is inserted into the sheath 2. The inner surface of the sheath 2 can have an extruded profile for mating with the outer surface of the elongated core 7. The outer surface of the elongated core has tabs **8** that mate tightly with the inner surface of the sheath in order to ensure that the elongated core does not rotate within the sheath. The force of the elongated core pushing against the inner surface of the sheath forms a seal between the elongated core 7 and the inner surface of the sheath 2. As shown, fluid inflow 5 channels and fluid outflow channels 6 are created between the outer sheath 2 and the elongated core 7.

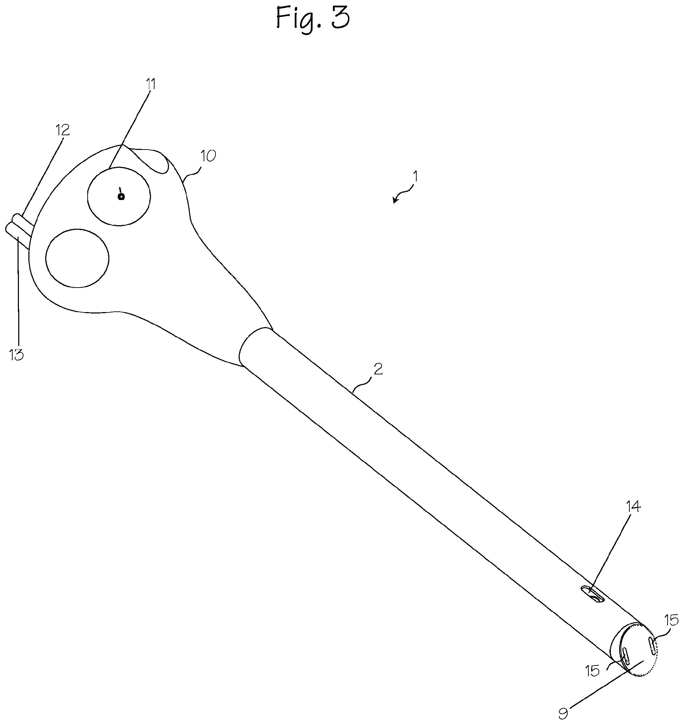

Figure 3 illustrates an arthroscope 1 having an optical cap **9.** The arthroscope has an ergonomic handle **10** for user comfort. The handle contains user control switches **11** that can provide focusing means for controlling the optical zoom of the system. At the distal end, the arthroscope also contains an electronics cable **12** and fluid inflow and outflow tubing **13.** Positioning of the electronics and fluid tubing eliminates clutter of conventional arthroscopes. The optical cap 9 is made of a plastic material and is located at the distal end of the arthroscope. The optical cap 9 may serve as the objective lens if one is not integrated into the imaging chip and associated package. Alternatively, the cap 9 may serve as a protective window, either optically clear or with optical modifying properties such as polarization or color filtering. The arthroscope also contains a fluid drain and sensor window **14.** A clear pocket flow of fluid flows axially to the system outflow from the distal end of the system. Drainage flows through openings **15** in the sheath 2. Flow in this direction creates a clear fluid pocket in front of the arthroscope where it is required the most.

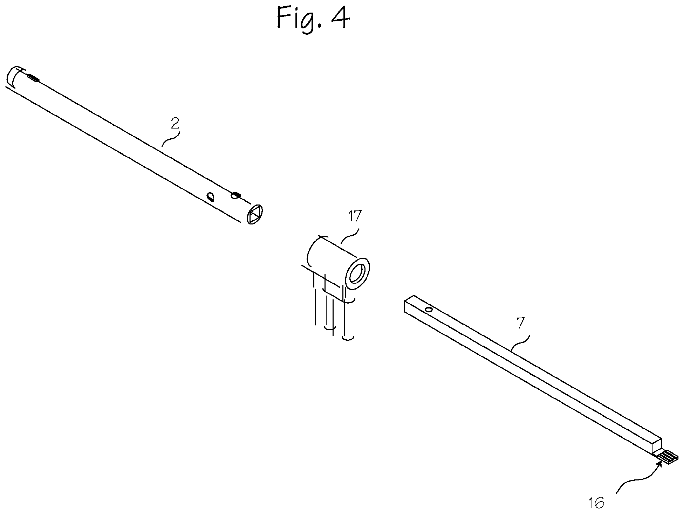

Figure 4 illustrates the features of the arthroscope 1 pulled apart. The distal end of the elongated core has a multifunction connector **16** for use with the video, pressure and temperature sensors. A round fluid sheath 2 is placed over the elongated core 7 and connected via a hub **17.** The hub can be coupled to a multi-channel fluid manifold. The outside diameter of the sheath closely matches the radial cross section of the elongated core to minimize the shape of the arthroscope. When engaged, the inner surface of the external sheath and the outer surface of the elongated core define a plurality of fluid channels extending longitudinally within the arthroscope. The fluid sheath can also have a rectangular radial cross section closely matching the radial cross section of the elongated core.

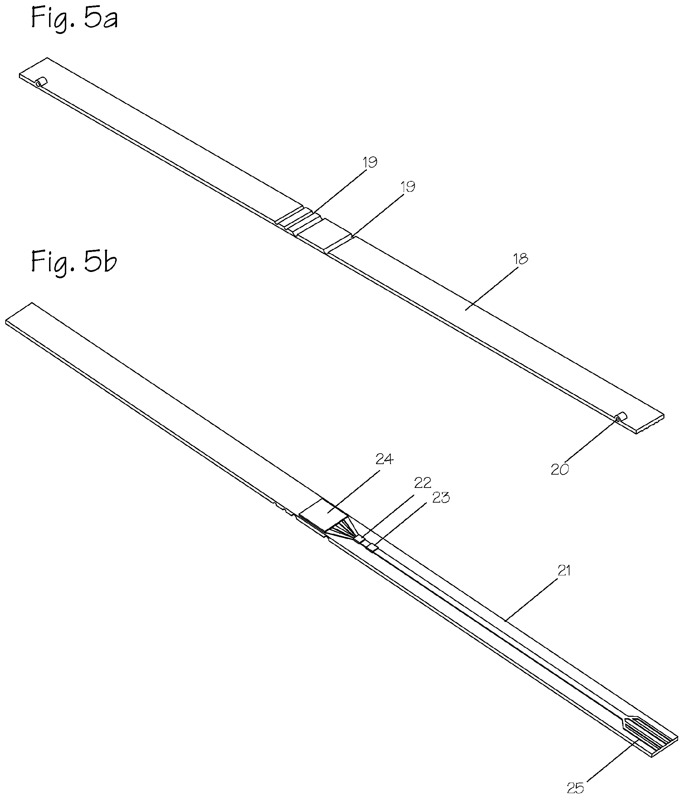

Figures 5a and 5b illustrate the elongated core 7 of the arthroscope before it is folded into its final configuration. Figure 5a illustrates the base of the elongated core. The elongated core is constructed onto a flat molded backing **18.** The backing 18 contains folds to create hinge points **19** that allow the backing to fold into the square configuration. The degree to which the folds are rotated allows the angle of the imaging chip to vary according to user preference. Pivot points **20** are contained at each end of the backing for connection of the top and bottom faces of the elongated core. Figure 5b illustrates the molded backing 18 with a flex circuit **21** laminated onto the molded hinge backing. The flex circuit 21 contains a pressure sensor **22** and a temperature sensor **23** as well as an imaging chip and its sensor module and associated lens **24.** The lens can be made of plastic or other similar material to assist in insulating the imaging chip and the inside electronics from damage. In addition, an edge connector **25** is contained on one end of the molded backing for connection to desired system input or power devices.

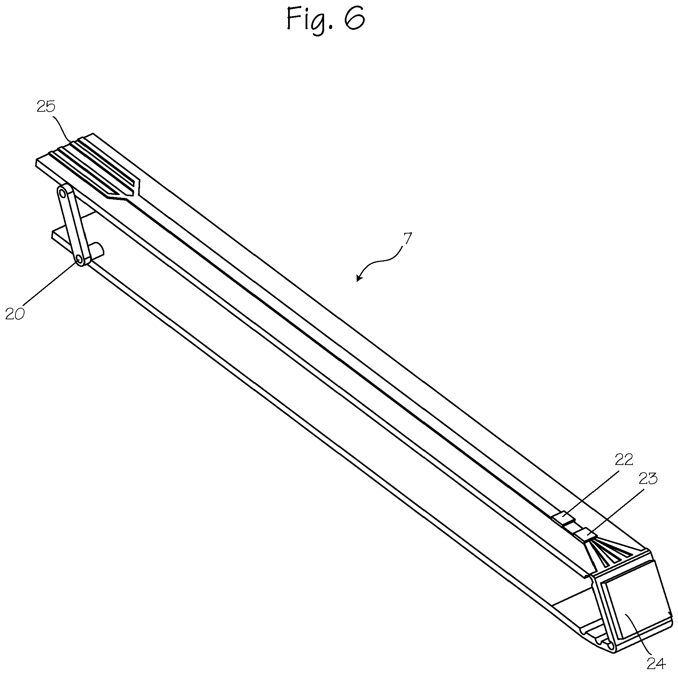

Figure 6 illustrates the elongated core of the arthroscope in its final folded configuration. At the distal end the elongated core houses the digital image CCD or CMOS chip and a sensor module 24 to enhance image magnification clarity and color. At the proximal end, the elongated core contains a multifunction edge connector 25 for use with the temperature 23 or pressure signal 22 connectors and to carry video signal. This elongated core is open on both sides. The elongated core 7 is formed by folding over the backing 18 and connecting the top and bottom backing faces at the pivot points 20. The elongated core shape is dictated by the combination of the square chip and associated chip package that are of pre-determined sizes and commercially available. The elongated core may contain one or multiple digital image chips within a single arthroscope. Longitudinal movement of a first face of the backing relative to a second face of the backing changes the angle of digital image CCD or CMOS chip to vary relative to the radial plane of the elongated core. The imaging end enables an indefinitely adjustable view angle from 0 degrees to 90 degrees in a single scope. The arthroscope can also accommodate for a 180 degree or retrograde view where the arthroscope has a flat top construction and a rotatable or living hinge rectangular arthroscope architecture. The elongated core 7 can be releasably mounted to a base such that the core can be sterilized and reused for a number of surgical procedures.

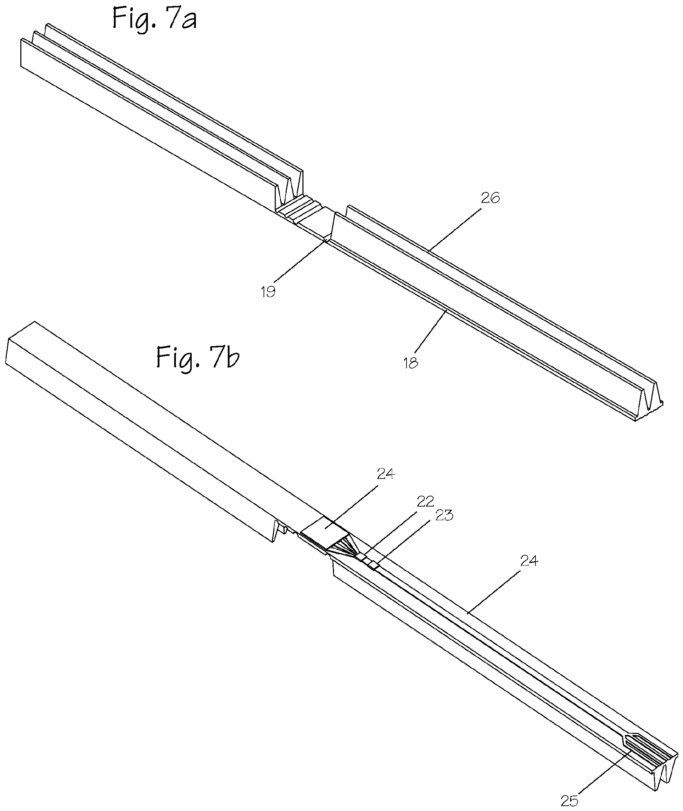

Figures 7a and 7b illustrate another elongated core 7 before it is folded into its final configuration. Figure 7a illustrates the backing 18 of the elongated core. The elongated core is constructed onto the molded backing 18 that contains protrusions **26** spaced apart at a predetermined distance. The protrusions on each face are matched to mate when in a folded configuration. When folded, the protrusions construct a solid elongated core. The elongated core has a square radial cross section with a proximal end, a distal end spaced from the proximal end for insertion into a body, a top surface, a bottom surface. The elongated core also has two opposite side surfaces adjacent to the top and bottom surfaces. At least one of the surfaces may contain a metal strip bonded to the top of the surface. The metal strip may be a spring steel or nickel-titanium alloy with a preformed radius of curvature. The metal alloy may alternatively be a malleable metal such as aluminum or may be a nickel-titanium (nitinol) alloy with a shape memory feature. The metal strip allows the elongated core to reliably bend in one plane of curvature. Where the memory backing is spring-steel or nitinol, it may bend to a shape if malleable, or may be made steerable with a nitinol shape-memory component.

The elongated core contains planes that provide structural rigidity to the elongated core. The protrusions can have a locking taper construction. In addition, the protrusions can be joined with an adhesive or can be welded together thermally or with ultrasonic welding techniques. The elongated core also contains an imaging device chip fitted at the distal end of the elongated core where the imaging surface is arranged in a viewing direction of the elongated core. In addition, the elongated core has an illumination source at the proximal end for illuminating a surgical site at which the arthroscopic sheath system is directed. The core backing 18 contains folds that create hinge points 19 to allow the backing to fold into a rectangle. Pivot points 20 are contained at each end of the backing for connection of the top and bottom faces of the elongated core. Figure 7b illustrates the molded backing 18 with a flex circuit 21 laminated onto the molded hinge backing. The flex circuit 21 contains the pressure and temperature sensors 22, 23 as well as the imaging chip and its associated LED package 24. In addition, the edge connector 25 is contained on one end of the molded backing.

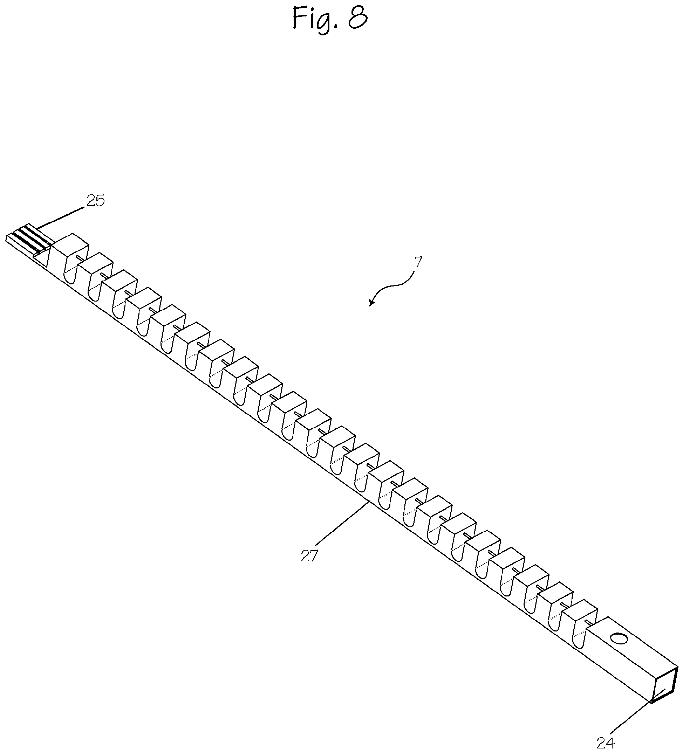

Figure 8 illustrates another elongated core configuration. The distal end of the elongated core 7 houses the digital image CCD or CMOS chip and sensor module 24. The distal end can also contain imaging modalities other then visible light devices such as ultrasonic transducers and optical coherence tomography (OCT) imagers in addition to the CCD and CMOS video imagers. At the proximal end, the elongated core contains a multifunction edge connector 25 for use with temperature or pressure signal connectors. The intermediate body of the elongated core is in the form of vertebrated or specifically profiled sections **27** located at a predetermined distance from each other to enhance steerability of the elongated core when inserted into the patient. The elongated core is transversely slotted along its entire length to form this configuration.

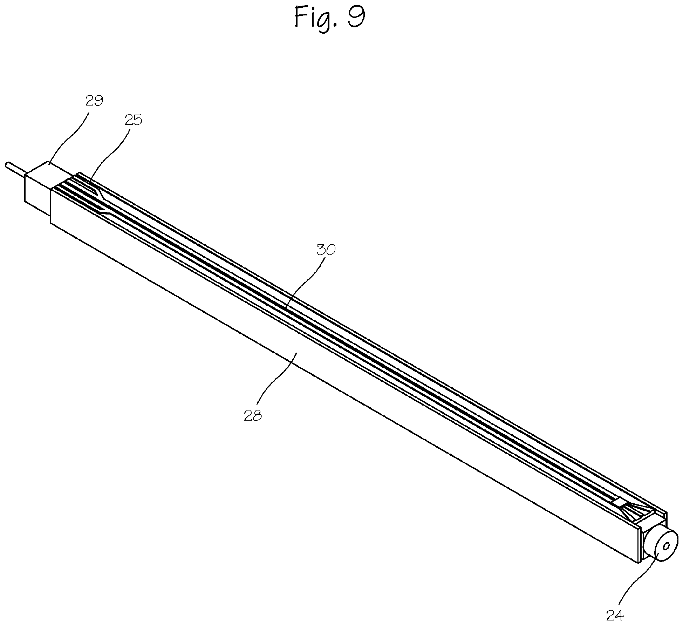

Figure 9 illustrates an elongated core with a square tube or solid mandrel for additional rigidity. The rectangular mandrel may serve as an illumination conduit. The assembly has an optically transparent light pipe center core **28** that allows light to pass through. Illumination light emanating from a light source apparatus passes through the transparent core, is converged by a lens, and falls on the opposing end surface of the illumination conduit. The illumination light is transmitted to the arthroscope over the illumination conduit, passes through the arthroscope, and is emitted forward through the distal end of the arthroscope. Thus, an object in the patient's body cavity is illuminated. An image represented by the light reflected from the illuminated object is formed by the arthroscope. A resultant object image is projected by the imaging means through the scope. The optically transmitting center core is a rectangular shaped housing or mandrel made of a molded plastic material that can transmit light from the proximal end and out of the distal end. The center core is made of any clear molded polycarbonate or acrylic plastic material that can be easily molded. The molded plastic mandrel has an LED illumination module **29** at the proximal end and the assembly circuitry **30** is wrapped around the center core. The edge connector 25 is also contained at the proximal end of the assembly. The chip imaging module 24 is contained at the distal end of the assembly. In addition, the distal end of the assembly serves as the transmitting end of the light pipe created by the center core. The advantage to the assembly is that it has a small cross-section, but is very robust and easy to use. The assembly is inexpensive to manufacture and provides adequate illumination to the arthroscope.

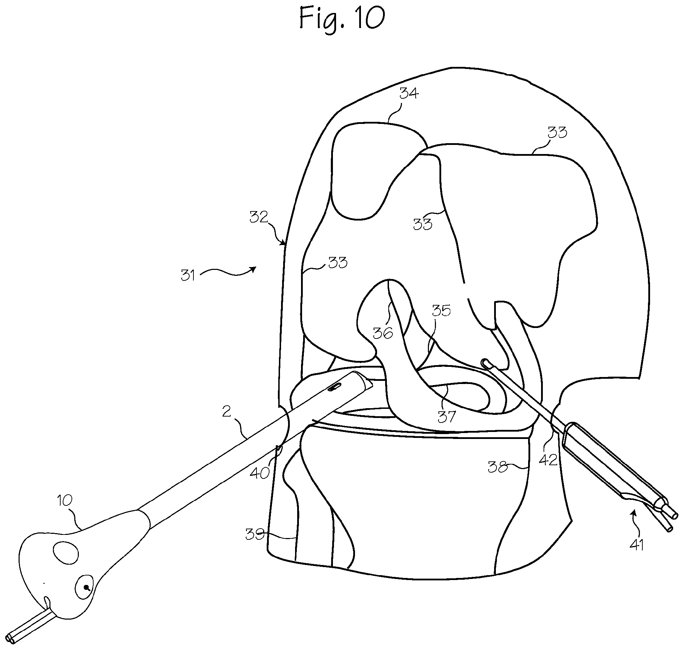

Figure 10 shows a method of performing arthroscopic surgery on a patient **31** using an arthroscope in an atraumatic sheath 2. Various anatomical landmarks in the patient's knee **32** are shown for reference, including the femur **33,** patella **34,** posterior cruciate ligament **35,** anterior cruciate ligament **36,** meniscus **37,** tibia **38** and fibula **39.** During surgery, the surgeon introduces the arthroscope into the knee via a first incision **40** in order to visualize the surgical field. A trimming instrument **41** is introduced through a second incision **42** to remove or trim tissue that the surgeon determines should be removed or trimmed.

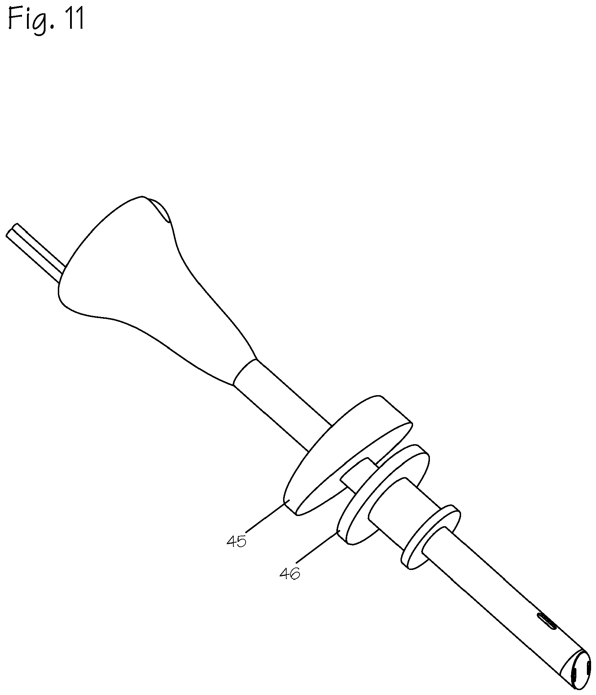

Figure 11 illustrates an arthroscope where the fluid management is contained in a grommet-type cannula. The arthroscope has an angle set collar **45** and an elastomeric portal cannula **46.** When the collar is not pressed to the elastomeric cannula, the scope set perpendicular to the portal. When the sleeve is pushed forward, the scope is angled in the portal. Where the collar is rotated, the arthroscope can be directed to an area of interest radially within the surgical space. The ability to translate, rotate and hold the scope can be accomplished with a ball gimbal or other similar means. This frees the hands of the surgeon to use their instruments rather than have to hold the arthroscope in position.

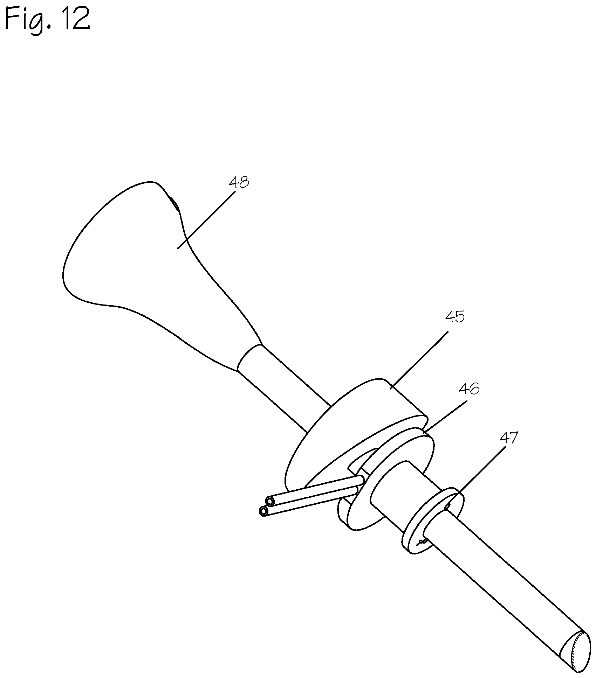

Figure 12 illustrates an arthroscope that can be used without requiring a user to hold it, providing the user the opportunity to use the arthroscope hands free. The arthroscope has an angle set collar 45, an elastomeric portal cannula 46 and a grommet cannula **47** to allow for fluid inflow and outflow through the grommet cannula. The fluid and gas management connections are removed from the arthroscope. The arthroscope also contains a wireless scope **48** that accommodates for multiple scopes to communicate on a network. This allows the arthroscope to be wireless and untethered by either wires or fluid tubes and instead to be aimed and held on a point of interest. This provides the advantage that the surgeon can use both hands while operating on a patient and can be useful in telemedicine applications. The arthroscope is wireless and can be networked together with a ZigBee, MESH or Bluetooth wireless network.

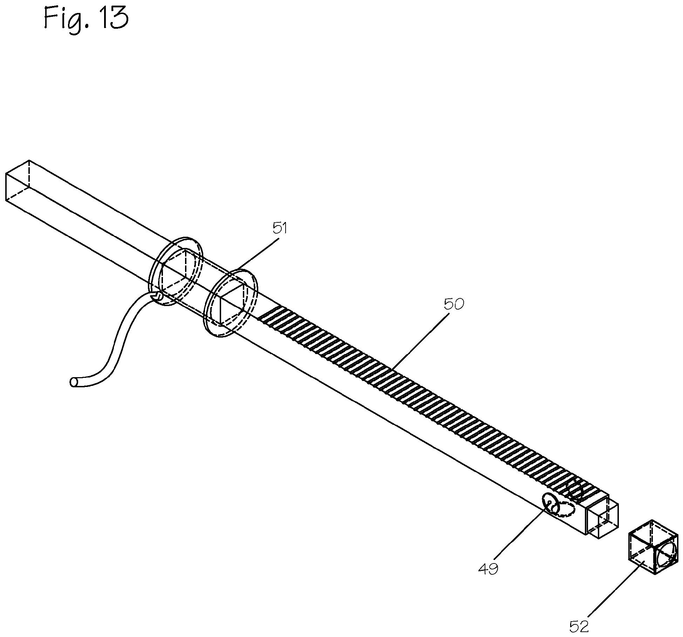

Figure 13 illustrates an arthroscope with a molded optical cap and 3-D positioning sensors. Spatial positioning and tracking sensors **49** can be attached to 3 of the 4 orthogonal sides of the arthroscope. These sensors can read optically, ultrasonically, or with an RFID system. The positioning and tracking system allows the arthroscope to be positioned accurately in space and can be used to guide surgical instruments and provide accurately guided cutting of tissue. In addition, due to the arthroscope's flat surface, a linear encoder **50** can be added to the arthroscope using circuit printing lithography techniques. This can be used to accurately gauge the depth of penetration of the scope into the surgical field. A reader **51** for the linear encoder is disposed within an access cannula. The data from the 3-D positioning and tracking means 49 and linear encoder 50 may be transmitted for display and processing either wired, or wirelessly. The 3-D and linear positioning encoders may be on two or more arthroscopes and can communicate and network together with a ZigBEE MESH network, Bluetooth 802.11 or other wireless protocol. The 3-D positioning and tracking can be useful for robotic surgery, virtual template aided surgery, augmented reality surgical visualization and high-risk surgery, or implant surgery where geometrically accurate cutting is essential to the proper alignment of a device such as an orthopedic implant. The system also has an optical cap **52** to protect the imaging chip from fluids. The cap is molded of acrylic, polycarbonate, or other appropriate optically clear plastic. The cap may be molded with a spherical lens, an aspheric lens, or a split stereoscopic lens that projects a binocular image on to the imaging chip. The central square rod may have a structural center core (e.g. stainless steel or titanium), to give the scope strength, and the perimeter of the rod may be clad with an optically clear light pipe of a light-transmitting plastic. The rod is illuminated at the proximal end with an LED light source or a fiber optic cable, and the light is transmitted through a pipe light, through the optical cap 52 out the distal end to illuminate the surgical field. On the perimeter, the optical cap may have a condensing lens feature, or a light diffusion means to tailor the illumination to the clinical needs of the surgeon. The system may be used with a fluid management sheath and means previously disclosed. Also the ability to build 100% polymer and non-ferrous arthroscope allows its use in radiology guided applications where the materials must be non-magnetic, such as under MRI applications.

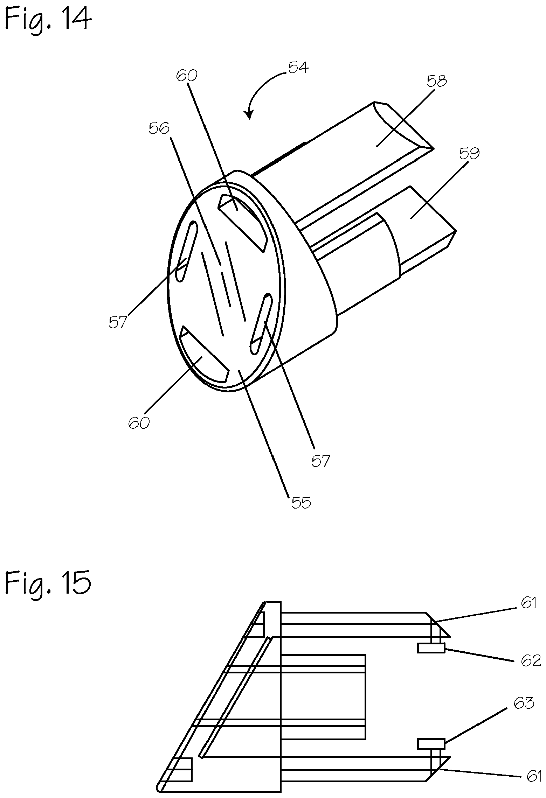

Figure 14 illustrates an optical cap **54** for use with an arthroscope system. The optical cap 54 has a cap face **55** that is optically clear and can have a plain transparent viewing window **56** or can be molded as a lens element. The optical cap is made of any optically clear biocompatible and moldable polymer, acrylic, optical polycarbonate, or other moldable optically clear plastic or non-conductive polymer. The optical cap may also be made of any optically clear silicon. The cap protects the distal end of the arthroscope from accidental damage and also emits light from the distal tip of the arthroscope. The cap face contains fluid channels **57** to allow the inflow of fluid into the surgical space. The cap also includes at least two inserted molded tubes or pipes **58, 59** for transport of light through the tubes with minimal loss of light. The tubes or pipes 58 can be light pipes or prisms that terminate at openings **60** at the distal end of the cap located on the cap face. Each light pipe contains a 45 degree prism or mirror **61** at the proximal end of the light pipe that directs light from the LEDs out of the end of the optical cap into the surgical space to facilitate the emission of light from the distal tip of the arthroscope. The cap also includes a first LED **62** and second LED **63** (shown in Figure 15) where each LED is located on the proximal end of the tubes or pipes 58. The LEDs may also be mounted on the flat, flexible circuit board laminated to the molded and foldable mandrel shown in Figure 7b.

The LEDs are preferably mounted in a direction orthogonal to the direction of emission of light from the distal end of the cap. The openings on the cap face allow for light output and also condense projected light from the LEDs. The cap may also include an indentation or molded-in lens to condense projected light. Light reflected off the prisms or mirrors is transmitted out of the front of the cap at the distal end of the arthroscope. The emitted light from the LEDs shine onto and reflect off of the prism or mirror and projects out from the distal tip of the arthroscope. The LEDs produce white light in the ultraviolet (UV) and the near infrared (NIR) ranges. Each light pipe is coated with an anti-dispersive coating or cladding to facilitate shining of light through the interior of the arthroscope.

Figure 15 shows a side view of an optical cap. The cap contains at least two light pipes 58, 59, each light pipe having an angled prism or mirror 61 at the proximal end of each light pipe. Each light pipe contains an LED positioned at the proximal end of the tube or pipe. Light emitted from the LEDs is reflected off of the mirror surfaces and projected out of the distal end of the arthroscope. The cap face is positioned over the distal tip of the arthroscope to protect the arthroscope from damage. The cap face can be of various viewing angles according to the surgical application viewing requirements. The cap face can have a 0degree or blunt face for use with hysteroscopes. A 30 degree tip angle can be used for arthroscopes while a 70 degree tip angle can be used for special viewing applications in arthroscopy. The distal end of the cap can also include a molded in lens on the larger optical cap surface or fly eye to allow light to be locally focused. The smaller molded in lens on the larger cap surface eliminates the need for secondary assembly procedures. Additionally, the arthroscopic system can include an accessory channel for delivering fluent medications, tissue adhesives, needles, anchors or sutures.

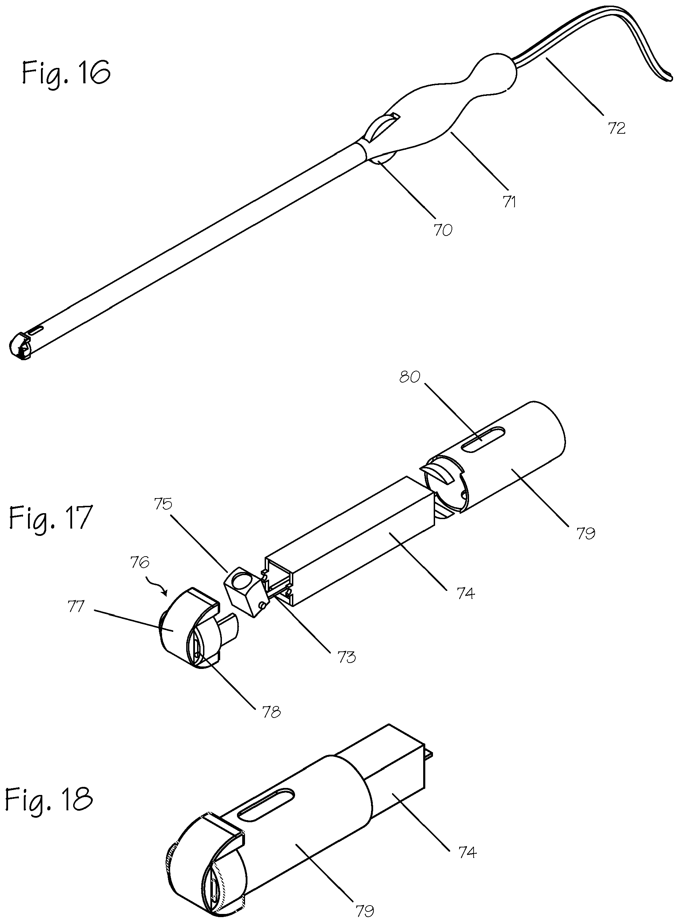

Figure 16 illustrates an arthroscope which provides for variable angle viewing. An angle of view selector **70** is embedded in the handle **71.** The variable angle of view may range from 0 to 90 degrees, and in particular, 0, 30 and 70 degrees are the most common viewing angles used in arthroscopic surgery. The distal tip provides a digital imager (detailed in Figure 17). This embodiment uses an articulated video module in conjunction with features previously described, including the square scope, the optical cap, fluid inflow and outflow, all within a minimal cross-section fluid tube. This embodiment also supports temperature and pressure sensing previously described. The device also contains a position encoder for the video module pushrod, which senses the viewing angle of the scope. The proximal end **72** provides for fluid, electronic and video connection, as well as flow, temperature and pressure measurements.

Figures 17 and 18 detail the distal tip of the instrument. Figure 17 shows the variable angle arthroscope of Figure 16. One way to achieve the variable angle viewing is to deflect the chip view plane to the desired angle. As shown, a chip camera module is actuated with a pushrod to provide for different viewing angles. The pushrod **73** enclosed within square support core **74** swings a pivoting camera **75** in positions ranging from 0 degrees to 90 degrees, in particular, 0, 30 and 70 degree viewing angles. Though a pushrod is shown, other mechanisms may be used for deflecting the imaging chip, such as a pullwire or jackscrew. A curved optical cap **76** allows for camera swing. A curved window **77** eliminates optical distortion. The cap contains fluid channels **78** to allow the inflow of fluid into the surgical space. Fluid flows out through sheath **79** via the outflow port **80.** Encoders may also be used to communicate to a video processor the angle of view. The view angle is displayed on a video monitor (not shown). A variable viewing angle allows the surgeon to change from a 0 to a 30 to a 70 degree angle of view without the need to change scopes. The surgeon can also pan the anatomy to get a better view of an arthroscopic workspace.

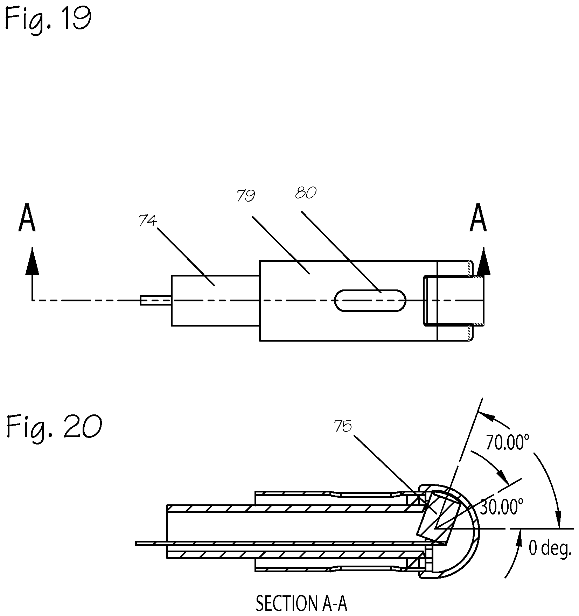

Figure 19 illustrates a top view of the distal tip of the arthroscope shown in Figure 16. Figure 20 shows a cross-sectional view of Figure 19. The variable angles of the imaging camera are shown.

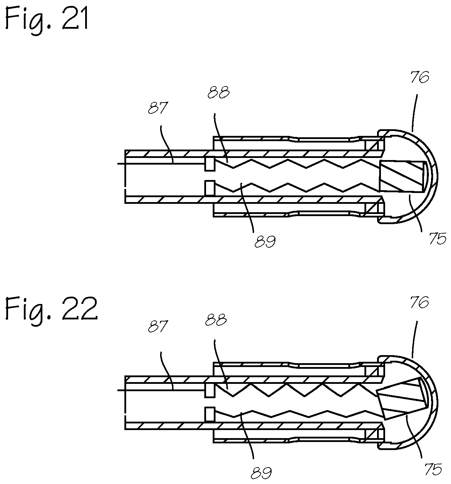

Other mechanisms may be employed to swing the pivoting camera. As shown in Figures 21 and 22, the camera may be deflected using a shape-memory nitinol wire or shape memory spring that is resistively heated to change its length or generate push or pull force, and thus provide the actuating force to deflect the camera. This has the advantage of being able to pan the camera without the need for a mechanical pullwire or pushrods mechanically actuated from the proximal handle. This allows for reliable actuation even when the shaft is bent at an acute angle, as the actuation is generated with an electric current via a wire **87.** The nitinol shape memory actuator **88** is trained such that, when heated, it shortens to pull on the pivoting camera 75 to deflect it (as shown in Figure 22). (Conversely, the actuator may be trained to lengthen upon heating, and push the camera to make it pivot.) A biasing spring **89** pulls the camera to "home" position when the resistive heating current stops and the nitinol actuator returns to its original longer shape (as shown in Figure 21).

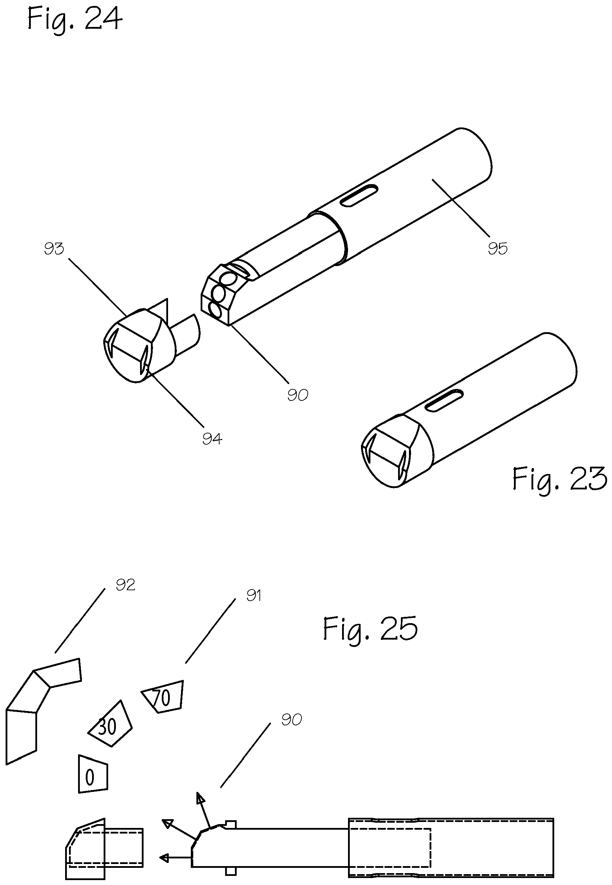

In the embodiment shown in Figures 23 and 24, a variable view angle is achieved by means of two or more imaging devices that are attached to planar surfaces at a pre-set viewing angles (item **90**). In the example shown, the endoscope has three pre-sets for 0 degrees, 30 degrees, and 70 degrees, as shown in Figures 24 and 25. The user selects the imaging device at the desired angle of view **91** (discrete angle view). The imaging devices may also operate simultaneously and the images stitched together in software to generate a panoramic view **92** (stitched view).

The digital scope has a clear optical cap. The cap may contain focusing lenses or optical filters including polarization, and wavelength filtering. The endoscope has a light source that illuminates the surgical field through the optical cap. The optical cap **93** with fluid management capability **94** covers the imaging devices, and a fluid management tube **95** covers the shaft of the endoscope. This produces two or more fluid channels with the outer tube circumscribing the rectangular core of the endoscope.

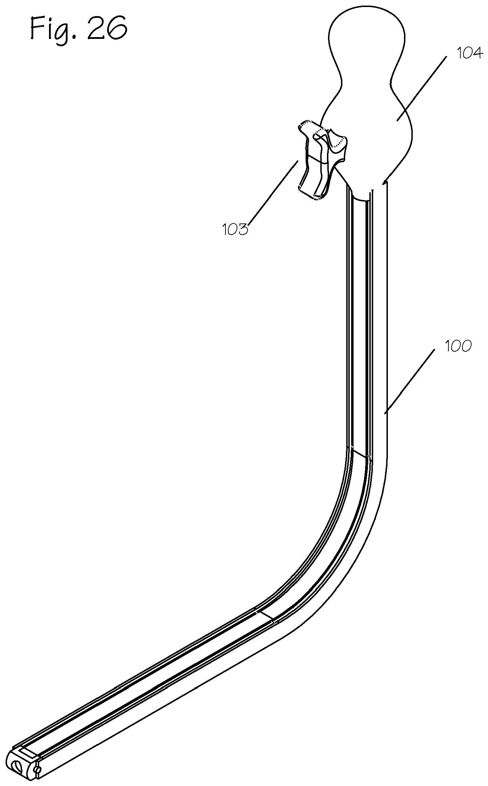

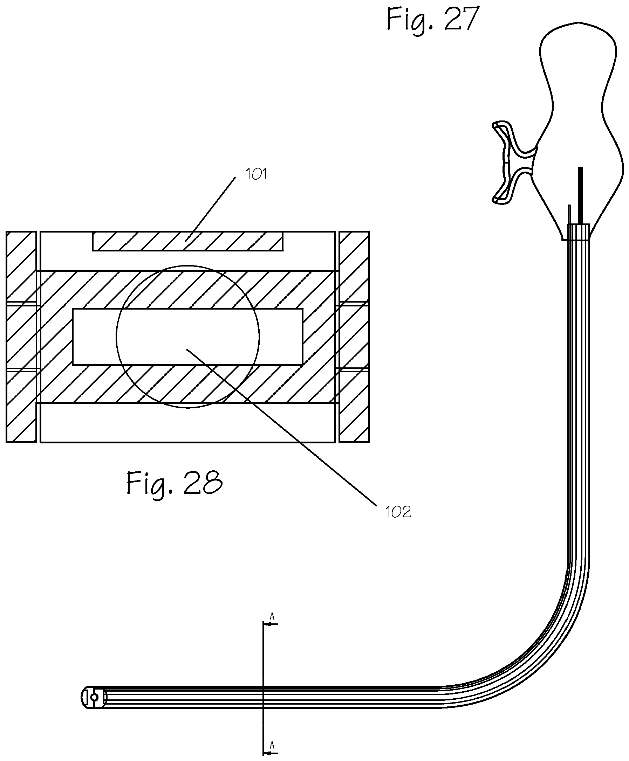

Figures 26, 27, and 28 illustrate an arthroscope which provides for variable angle viewing in a bent shaft **100** configuration. This embodiment allows the viewing angle of the camera at the distal end to be changed, and allows the camera shaft to be bent at an angle. The pullwire **101** to actuate the view angle lie in tracks in the i-beam shaped extrusion, and the wiring for the camera module goes through the conduit track **102** in the center of the extrusion. This allows enough slack in the data wires to allow movement of the video module at the distal end. The angle of view is controlled with a steering device **103** on the handle **104.** The angled shaft may be either rigid and fixed, or the shaft may be malleable, and set to a desired angle by the user. The angled shaft embodiment may have a fluid management tube and an optical cap with a fluid management means as previously described.

The digital scope embodiments lend themselves to the ability to bend the scope shaft to gain access to anatomy not accessible with a straight rigid scope. Because of the architecture of the scope, the endoscope has the ability to have a variable angle of view, with the scope shaft bent at an angle, as well as all of the other capabilities previously noted. The angle of the shaft may either be pre-molded and fixed, or malleable and the bend angle and radius selected by the user.

In use, a surgeon inserts the elongated core into the sheath of corresponding size. The elongated core creates the most space efficient configuration in that the insertion of the elongated core into the smallest complimentary circular shaped sheath eliminates wasted space. In addition, the arthroscope allows for an efficient clear pocket view flow of fluid inflow and outflow to create a clear field of view for the surgeon. Also, the arthroscope can be used as a retractor once inserted in the patient.

Alternatively, in use, a surgeon inserts the optical cap over the arthroscopic system in viewing direction of the arthroscopic system before inserting the entire system into a patient. The LEDs are activated so that light is emitted from the LED at the proximal end of the light pipes. Light emitted from the LEDs is reflected off the 45 degree prism or mirror of the light pipes and projects from the distal tip of the arthroscope. The position of the LEDs allows irrigation fluid passing through the inflow channel to cool off the imaging chips and imaging sensor, resulting in less image noise. In addition, because the cap is constructed of a non-conductive polymer, the imaging chip is isolated and therefore protected from stray RF current. The optical cap insulates the imaging chip located on the elongated core of the arthroscopic system from damage from radiofrequency surgical devices commonly used in surgery.

The arthroscope architecture allows the largest possible elongated core to be used in the smallest scope sheath. The elongated core dimensions are matched to the scope sheath to accommodate for a low profile system. Further, the arthroscope allows for a flat rectangular scope with a panoramic view, as well as 3-D viewing where two chips are placed side-by-side. In addition, multiple arthroscopes can be used at the same time in a single application system. Two or more arthroscopes can be aimed at a particular area of interest and the user can switch between the arthroscopic cameras with a selector device such as a footswitch. This frees up the user's hands to focus on other surgical instruments such as an arthroscopic shaver or stitcher without requiring use of his hands to hold the camera in place. The multiple arthroscope configuration can be held in place by a portal plug device. The plug can have an angled foot and be rotated to place the arthroscope at the desired angle. A plug can anchor the surgical portal as well as provide a means for sealing the portal to prevent leakage of fluid or gas. The plug can have a square inner lumen to seal against a square arthroscope that does not have an outer round sheath. The two or more cameras can be switched back and forth to cover multiple locations from a central console. The cameras can be of different focal lengths or have imaging capabilities such as narrow-light band imaging, near infra-red, optical coherence tomography miniature radiology device or other non visible light imaging modalities.

The imaging chip that generates video may also be incorporated to have the capability to pick up infrared. Thus, a chip camera can read temperature in a joint without the need for a thermocouple. The chip is programmed to dedicate a small area of the chip as an "area of interest" and the IR on the surface of what the chip is looking at is read, and interlaced between video frames, thus reading temperature. This data can be gathered simultaneously with recording a video image, and does not interfere with capturing the video image.

In conjunction with the fluid temperature management platform, this chip can read temperature, and this inter-articular temperature reading may be used to control a fluid pump that incorporates cooling (to prevent RF heat damage to tissue) and warming (to prevent patient hypothermia during arthroscopy). This could be in addition to the scope having an on-board MEMS pressure sensor for accurate inter-articular pressure measurement. These data may be transmitted to the irrigation pump temperature and pressure control unit by means of a wired connection or a wireless connection such as Bluetooth or 802.11.

The word arthroscope used herein includes a family of instruments, including endoscopes, laparoscopes and other scopes. The scopes may use rod optics, fiber optics, distally mounted CCD chips, or other optical systems. Thus, while the preferred embodiments of the devices and methods have been described in reference to the environment in which they were developed, they are merely illustrative of the principles of the inventions. Other embodiments and configurations may be devised without departing from the scope of the appended claims.

## Claims

1. An arthroscope, said arthroscope comprising:
a sheath (2) having an outer diameter and an inner diameter, the inner diameter having an extruded profile and having an inner surface and an outer surface;
an molded elongated core (7), constructed of a flat molded backing (18) folded onto a square configuration, the elongated core (7) having an inner and an outer surface, a square radial cross section, a proximal end, a distal end spaced from the proximal end for insertion into a body, a first surface with at least one hinge point (19), and a second surface with at least one hinge point (19), the molded backing being folded on the hinge points (19); and
an imaging device chip (3) fitted at the distal end of the elongated core (7) having an imaging surface arranged in a viewing direction of the arthroscope;
wherein the longitudinal movement of the first surface of the elongated core (7) relative to the second surface of the elongated core (7) changes the angle of the imaging chip (3) relative to a radial plane of the elongated core (7);
wherein when the elongated core (7) is inserted into the sheath (2), the inner surface of the sheath (2) and the outer surface of the elongated core (7) define an inflow channel (6) extending longitudinally within the arthroscope and an outflow channel (5) extending longitudinally within the arthroscope, so that fluid may enter the inflow channel (6) and exit through the outflow channel (5) and come behind the distal end of the arthroscope.

2. The arthroscope of claim 1 wherein the elongated core (7) further comprises protrusions (8) on the first and second surfaces spaced apart at a predetermined distance and matched to mate with the extruded profile of the inner diameter of the sheath.

3. The arthroscope of claim 1 further comprising two opposite side surfaces adjacent to the first and second surfaces.

4. The arthroscope of claim 1 further comprising:
an illumination source at the proximal end of the arthroscope for illuminating a surgical site at which the arthroscope is directed.

5. The arthroscope of claim 1 wherein the elongated core (7) further has protrusions spaced apart at a predetermined distance that are matched to mate with the extruded profile of the inner diameter of the sheath (2).

6. The arthroscope of claim 1wherein the imaging chip (3) is adapted to change angle positions as the hinge points (19) of the first and second surfaces are adjusted.

7. The arthroscope of claim 1 wherein the sheath inner diameter closely matches the square radial cross section dimension of the elongated core (7).

8. The arthroscope system of claim 1 further comprising a fluid manifold operably connected to the external sheath, said manifold comprising a plurality of fluid pathways communicating with the inflow and outflow channels.

## Patentansprüche

1. Ein Arthroskop, aufweisend:
eine Hülle (2), welche einen äußeren Umfang und einen inneren Umfang aufweist, wobei der innere Umfang ein extrudiertes Profil aufweist und eine innere Oberfläche und eine äußere Oberfläche aufweist;
einen geformten länglichen Kern (7), welcher aus einer flachen geformten Grundschicht (18) konstruiert ist, welche in eine quadratische Konfiguration gefaltet ist, wobei der längliche Kern (7) eine innere und eine äußere Oberfläche, einen quadratischen radialen Querschnitt, ein proximales Ende, ein distales Ende, welches in einem Abstand von dem proximalen Ende angeordnet ist zum Einsetzen in einen Körper, eine erste Oberfläche mit mindestens einem Gelenkpunkt (19), und eine zweite Oberfläche mit mindestens einem Gelenkpunkt (19) aufweist, wobei die geformte Grundschicht an den Gelenkpunkten (19) gefaltet ist; und
ein Bildgebungsgerätechip (3), welcher an dem distalen Ende des länglichen Kerns (7) montiert ist und eine Bildgebungsoberfläche aufweist, welche in eine Blickrichtung des Arthoroskops angeordnet ist;
wobei longitudinales Bewegen der ersten Oberfläche des länglichen Kerns (7) relativ zur zweiten Oberfläche des länglichen Kerns (7) den Winkel des Bildgebungschips (3) relativ zu einer radialen Fläche des länglichen Kerns (7) verändert;
wobei der längliche Kern (7) in der Hülle (2) eingesetzt ist, die innere Oberfläche der Hülle (2) und die äußere Oberfläche des länglichen Kerns (2) einen Zufluss-Kanal (6), welcher sich longitudinal innerhalb des Arthroskops erstreckt, und einen Abfluss-Kanal (5) definieren, welcher sich longitudinal innerhalb des Arthroskop erstreckt, so dass ein Fluid durch den Zufluss-Kanal (6) zufließen und durch den Abfluss-Kanal (5) abfließen und hinter das distale Ende des Arthroskops gelangen kann.

2. Arthroskop gemäß Anspruch 1, wobei der längliche Kern (7) ferner Vorsprünge (8) auf der ersten und zweiten Oberfläche aufweist in einem vorbestimmten Abstand zueinander, welche so angepasst sind, dass sie sich mit dem extrudierten Profil auf dem inneren Umfang der Hülle (2) zusammenfügen.

3. Arthroskop gemäß Anspruch 1, ferner aufweisend zwei gegenüberliegende, an die erste und zweite Oberfläche angrenzende Seitenflächen.

4. Arthroskop gemäß Anspruch 1, ferner aufweisend:
eine Beleuchtungsquelle am proximalen Ende des Arthroskops zum Beleuchten einer Operationsstelle, auf welche das Arthroskop ausgerichtet ist.

5. Arthroskop gemäß Anspruch 1, wobei der längliche Kern (7) ferner in einem vorbestimmten Abstand zueinander angeordnete Vorsprünge aufweist, welche so angepasst sind, dass sie sich mit dem extrudierten Profil des inneren Umfangs der Hülle (2) zusammenfügen.

6. Arthroskop gemäß Anspruch 1, wobei der Bildgebungschip (3) eingerichtet ist, Winkelpositionen zu ändern, wenn die Gelenkpunkte (19) der ersten und zweiten Oberfläche angepasst werden.

7. Arthroskop gemäß Anspruch 1, wobei der innere Umfang der Hülle (2) weitgehend dem den quadratischen radialen Querschnitt des länglichen Kerns (7) entspricht.

8. Arthroskop gemäß Anspruch 1, ferner aufweisend einen Fluid-Verteiler, welcher wirkfähig mit der äußeren Hülle verbunden ist, wobei der Verteiler eine Vielzahl von Fluidpfaden aufweist, welche mit dem Zufluss- und Abluss-Kanal verbunden sind.

## Revendications

1. Arthroscope, ledit arthroscope comportant:
une gaine (2) ayant un diamètre extérieur et un diamètre intérieur, le diamètre intérieur ayant un profilé extrudé et ayant une surface interne et une surface externe;
une partie centrale allongée moulée construit d'un support plat moulé (18) replié sur une configuration carrée, la partie centrale allongée (7) ayant une surface interne et externe, une section transversale radiale carrée, une extrémité proximale, une extrémité distale espacée de l'extrémité proximale pour l'insertion dans un corps, une première surface avec au moins un point de charnière (19) et une seconde surface avec au moins un point de charnière (19), le support moulé étant replié sur les points de charnière (19); et
une puce de dispositif d'imagerie (3) fixée à l'extrémité distale de la partie centrale allongée (7) ayant une surface d'image agencée dans une direction de visée de l'arthroscope;
le mouvement longitudinal de la première surface de la partie centrale allongée (7) relative à la seconde surface de la partie centrale allongée (7) changeant l'angle de la puce d'imagerie (3) relative au plan radial de la partie centrale allongé (7);
lorsque la partie centrale allongée (7) est inséré dans la gaine (2), la surface interne de la gaine (2) et la surface externe de la partie centrale allongée (7) définissant un canal d'entrée (6) s'étendant longitudinalement dans l'arthroscope et un canal de sortie (5) s'étendant longitudinalement dans l'arthroscope de sorte que du fluide peut entrer dans le canal d'entrée (6) et sortir par le canal de sortie (5) et arrive derrière l'extrémité distale de l'arthroscope.

2. Arthroscope selon la revendication 1, la partie centrale allongée (7) comportant en outre des protubérances (8) sur la première et la seconde surface espacées à une distance prédéterminée et adapté pour s'apparier avec le profilé extrudé du diamètre intérieur de la gaine.

3. Arthroscope selon la revendication 1, comportant en outre deux surfaces latérales opposées adjacentes à la première et la seconde surface.

4. Arthroscope selon la revendication 1, comportant en outre:
une source d'illumination à l'extrémité proximale de l'arthroscope pour illuminer un site opératoire sur lequel l'arthroscope est dirigé.

5. Arthroscope selon la revendication 1, la partie centrale allongée (7) en outre ayant des protubérances espacées à une distance prédéterminée adaptées pour s'apparier avec le profilé extrudé du diamètre intérieur de la gaine (2).

6. Arthroscope selon la revendication 1, la puce d'imagerie (3) étant adaptée pour changer des positions d'angle lorsque les points de charnière (19) de la première et la seconde surface sont ajustés.

7. Arthroscope selon la revendication 1, le diamètre intérieur de la gaine correspondant de près à la dimension de la section transversale radiale carrée de la partie centrale allongée (7).

8. Système arthroscopique selon la revendication 1, comportant en outre un collecteur de fluide relié de manière opérationnelle à la gaine externe, ledit collecteur comportant une pluralité de chemins de fluide communiquant avec les canaux d'entrée et de sortie.
